# EUROPÄISCHE PATENTANMELDUNG

(11) **EP 3 120 880 A1**
(43) Veröffentlichungstag der Anmeldung: **25.01.2017**
(21) Anmeldenummer: 15177575.6
(22) Anmeldetag: 20.07.2015
(51) Int. Cl.: A61M 1/10, A61M 1/12

(54) **IMPLANTIERBARES PUMPENSYSTEM SOWIE VERFAHREN ZUM EINBRINGEN EINES PUMPENSYSTEMS AN EINEN EINSATZORT**

(71) Anmelder: Berlin Heart GmbH, 12247 Berlin (DE); Universität Zürich, 8006 Zürich (CH)
(72) Erfinder: OPFERMANN, Dr., Ulrich Tim, 12247 Berlin (DE); ARNDT, Dr., Andreas, 12555 Berlin (DE); FALK, Prof. Dr., Volkmar, 10115 Berlin (DE)
(74) Vertreter: Pfenning, Meinig & Partner mbB

(57) **Zusammenfassung**

Die Erfindung bezieht sich auf ein implantierbares Pumpensystem zur Förderung von Blut innerhalb eines Patientenkörpers mit einer Blutpumpe (2, 3, 8), die eine Flüssigkeit in einer Axialrichtung (7) fördert und die einen rotierend antreibbaren Rotor (3) sowie ein den Rotor umgebendes Pumpengehäuse (2) aufweist, sowie ein Stützrohr (6), in dem das Pumpengehäuse (2) angeordnet und gehalten ist, wobei zwischen dem Stützrohr und dem Pumpengehäuse ein Ringspalt (33) gebildet ist. Auf diese Weise wird durch die Kombination einer Strömung durch das Pumpengehäuse (2) einerseits und den Ringspalt (33) andererseits eine nahezu physiologische Blutströmung ermöglicht.

## Beschreibung

Die vorliegende Erfindung liegt auf dem Gebiet der Mechanik und ist mit besonderem Vorteil in der Medizintechnik im Bereich implantierbarer Pumpen einsetzbar.

Mit besonderem Vorteil ist die Erfindung im Bereich der Blutpumpen einsetzbar, die die Blutförderung im Kreislauf eines Lebewesens innerhalb von Blutgefäßen und gegebenenfalls zwischen einem Herz und angrenzenden Blutgefäßen unterstützen.

Es sind implantierbare Blutpumpen zur Unterstützung des linken Herzventrikels (Left Ventricular Assist Devices, LVAD) bekannt, die innerhalb des Herzbeutels (intraperikardiale Platzierung) von außen mittels einer Kanüle an den linken Ventrikel angeschlossen werden und sogar ganz oder teilweise in den linken Ventrikel hineinragen (intraventrikuläre bzw. partiell intraventrikuläre Platzierung). Die Pumpe entnimmt dem linken Ventrikel das Blut und fördert es üblicherweise über eine relative lange Auslasskanüle in die Aorta. Diese Pumpen bieten den Vorteil einer adäquaten Förderleistung bei einem relativ niedrigen Grad der Blutschädigung. Nachteilig ist die invasive Implantationsweise, die eine mediane Sternotomie oder mindestens eine Thorakotomie erfordert. Ebenfalls nachteilig ist die verhältnismäßig große Blutkontaktfläche, verbunden mit einer Anregung des Gerinnungssystems. Intraventrikuläre oder partiell intraventrikuläre Pumpen haben außer einer relativ großen Berührungsfläche mit dem Blut zudem das Problem, dass eine starre Verankerung der Pumpe im Apex bei Relativbewegungen der Pumpe und der Kanüle zum Endokard, zur Aortenklappe und zur Innenwand der Aorta Verletzungen, Entzündungsreaktionen oder Gewebewucherungen induzieren kann. Auch hier können thromboembolische Komplikationen die Folge sein.

Vor dem Hintergrund des Standes der Technik liegt der vorliegenden Erfindung die Aufgabe zugrunde, ein stabiles und zuverlässiges implantierbares Pumpensystem zu schaffen, bei dem die mit der zu fördernden Flüssigkeit in Berührung kommende Fläche klein gehalten und bei dem beim Einsatz als Herzunterstützungspumpe eine möglichst physiologisch kompatible Blutströmung erzeugt oder zugelassen werden kann.

Die Aufgabe wird mit den Merkmalen der Erfindung gemäß den Patentansprüchen gelöst.

Die Erfindung bezieht sich somit konkret auf ein implantierbares Pumpensystem zur Förderung von Blut innerhalb eines Patientenkörpers mit einer Blutpumpe, die eine Flüssigkeit in einer Axialrichtung fördert und die einen rotierend antreibbaren Rotor sowie ein den Rotor umgebendes Pumpengehäuse aufweist.

Gemäß der vorliegenden Erfindung ist vorgesehen, dass das Pumpensystem zusätzlich ein Stützrohr aufweist, in dem das Pumpengehäuse angeordnet und gehalten ist, wobei zwischen dem Stützrohr und dem Pumpengehäuse ein Ringspalt gebildet ist.

Durch die erfindungsgemäße Konstruktion kann einerseits im Inneren des Pumpengehäuses insbesondere durch einen Rotor Flüssigkeit, also insbesondere Blut, in einer Axialrichtung gefördert werden. Diese Blutförderung kann weitgehend unabhängig von dem physiologisch aufgebauten Blutdruck, also beispielsweise von der Phase des Herzrhythmus, gefördert werden.

Zusätzlich ist gemäß der erfindungsgemäßen Konstruktion jedoch durch den Ringspalt zwischen dem Pumpengehäuse und dem Stützrohr eine weitere Strömung möglich, die von der unmittelbaren Förderung durch die Blutpumpe unabhängig ist. Der Ringspalt kann insbesondere in radialer Richtung wenigstens 0,3 mm, insbesondere wenigstens 0,5 mm, vorteilhaft 1,5 bis 3,5 mm breit sein. Diese Blutströmung durch den Ringspalt kann beispielsweise dem durch die Herzaktivität geförderten Blutstrom entsprechen. Dadurch wird die Herzaktivität unabhängig von der maschinellen Unterstützung nicht behindert, und dem durch die Blutpumpe erzeugten Blutstrom wird ein physiologisch natürlich gesteuerter Blutstrom überlagert. Dies kann insbesondere bezüglich der Pulsation des Blutstroms sehr nützliche Effekte haben, da das Entstehen einer stationären Strömung verhindert, entsprechende nichtstationäre Strömungszustände erzeugt und damit Totwassergebiete der Blutströmung vermieden werden. Auch die Funktion von Blutdrucksteuerungsmechanismen im Patientenkörper wird dadurch weitgehend intakt gehalten. Bei einer hohen Förderleitung des Herzens kann ein Teil des vom Herzen geförderten Volumenstroms durch diesen Ringspalt als Bypass zur Pumpe nutzen. Dies bietet weiterhin den Vorteil, dass bei einem etwaigen Ausfall der Pumpe dieser Strömungspfad weiterhin zur Verfügung steht.

Der Ringspalt zwischen dem Stützrohr und dem Pumpengehäuse kann als Kreisring ausgeformt sein, jedoch kann auch die Blutpumpe in Radialrichtung gegenüber der Mittelachse des Stützrohrs verschoben sein, so dass ein unregelmäßig geformter Ringspalt entsteht.

Die Blutpumpe selbst kann starr, d. h. nicht radial komprimierbar, oder aber radial komprimierbar ausgebildet sein. Im Fall einer nichtkomprimierbaren Blutpumpe wird zum Implantieren das Stützrohr so weit radial komprimiert, dass es das Pumpengehäuse der Blutpumpe möglichst eng umgibt. Ist die Blutpumpe selbst komprimierbar, so kann das Stützrohr weiter radial komprimiert werden, so dass beim Implantieren sowohl das Stützrohr als auch das Gehäuse der Blutpumpe und gegebenenfalls auch der Rotor der Blutpumpe radial komprimiert sind. Nach dem Herausschieben aus dem Hohlkatheter beim Implantiervorgang wird dann das Stützrohr weiter radial expandieren als das Pumpengehäuse der Blutpumpe, so dass ein entsprechender Ringspalt zwischen dem Stützrohr und dem Pumpengehäuse entsteht.

Um zu verhindern, dass das Blut, das die Pumpe und/oder das Herz fördern, durch den Ringspalt zurückfließt, kann der Ringspalt eine Ventileinrichtung aufweisen, die in einer ersten Flussrichtung eine Flüssigkeit in Axialrichtung den Ringspalt passieren lässt und den Ringspalt bezüglich der entgegengesetzten zweiten Flussrichtung sperrt. Durch eine derartige Ventileinrichtung wird sichergestellt, dass bei einer Platzierung des Pumpensystems im Bereich der Aortenklappe Blut nur in eine Richtung, und zwar aus dem linken Ventrikel heraus, strömen, nicht jedoch über den Ringspalt in dieses zurückströmen kann. Aus diesem Grund verschließt die Ventileinrichtung eine Flussrichtung im Bereich des Ringspalts. Die Ventileinrichtung kann als Rückschlag-Klappenventil ausgebildet sein.

Es ist jedoch auch vorteilhaft denkbar, dass eine zweite Ventileinrichtung den Querschnitt des Pumpengehäuses freigibt bzw. sperrt. In diesem Fall steuert diese Ventileinrichtung ausschließlich die physiologische Strömung innerhalb der Blutpumpe, selbst dann, wenn die Blutpumpe das Druckgefälle zwischen der Herzkammer und der Aorta nicht aufrechterhält, was bei einem etwaigen Defekt der Pumpe oder bei einer zu geringen Förderleitung der Pumpe der Fall sein kann, und verhindert damit die Rückströmung von Blut in die Herzkammer durch das Pumpengehäuse.

Es ist auch denkbar, auf jegliche Ventileinrichtung zu verzichten und durch vorteilhafte Ausgestaltung der Strömungscharakteristik im Abstromgebiet der Pumpe einen Rückstrom bzw. eine nennenswerte Rückströmung durch den Ringspalt zu verhindern.

Das Stützrohr kann grundsätzlich, wie oben beschrieben, in Radialrichtung expandierbar sein. Dies kann bei verschiedenen Implantationsmethoden die Platzierung erleichtern.

Die Pumpe samt Stützrohr ist für die Implantation im Bereich der Aortenklappe vorgesehen und soll vorteilhafterweise durch den Apex des linken Ventrikels bis in den Bereich der Aortenklappe vorgeschoben und dort verankert werden.

Um diese Verankerung zu ermöglichen, sieht eine vorteilhafte Ausgestaltung des Stützrohrs vor, dass dieses als Stent ausgebildet ist. Dieser kann vorteilhaft mit Halteankern versehen sein, die bei der Implantation für eine axiale Positionierung und Verankerung des Stützrohrs im Aortenklappensinus sorgen können, während die Stützdrähte des Stents, die grundsätzlich beim Vorschieben des Stents aus dem Hohlkatheter nach den Halteankern freigegeben werden, ein Andrücken der Aortenklappentaschen an die Aortenwand und eine Verklemmung des Stützrohrs in der Aorta bewirken.

Die Halteanker und Stützdrähte sind üblicherweise als federelastische Drähte, insbesondere aus einem Gedächtnislegierungsmaterial wie beispielsweise Nitinol, gefertigt.

Vorteilhaft kann das Stützrohr zudem als faltbares Drahtgeflecht ausgebildet sein oder ein solches faltbares Drahtgeflecht aufweisen. Durch eine derartige Konstruktion wird das Stützrohr leicht und reversibel radial komprimierbar und expandierbar. Zudem kann ein solches Drahtgeflecht durch elastisches Verklemmen an der Aortenwand leicht fixiert werden.

Die Blutpumpe kann gemäß der Erfindung vorteilhaft derart ausgestaltet sein, dass sie einen in Axialrichtung Blut fördernden Rotor aufweist, der im Pumpengehäuse in wenigstens einem Lager, insbesondere in zwei Lagern, drehbar gelagert ist. Entsprechende Rotationslager, die vorteilhaft als Gleitlager (hydrodynamische Lager), Kontaktlager, magnetische Lager oder einer Kombination aus diesen ausgebildet sind, können beispielsweise an einer Einström- und/oder Ausströmöffnung des Pumpengehäuses vorgesehen sein.

Ein derartiger Rotor einer Blutpumpe kann beispielsweise mittels einer antreibbaren biegsamen Welle angetrieben werden, die durch einen Hohlkatheter beispielsweise innerhalb der Aorta oder eines anderen Blutgefäßes bis zu einer Schleuse und von dort bis zu einem Motor verläuft, der innerhalb oder außerhalb des Patientenkörpers angeordnet sein kann.

Es kann jedoch auch vorteilhaft vorgesehen sein, dass die Blutpumpe im Bereich des Stützrohres, insbesondere innerhalb des Stützrohres, einen Motor aufweist. Dazu kann der Motor unmittelbar am Rotor vorgesehen sein. Ein solcher Motor kann beispielsweise als Elektromotor oder auch als Turbine ausgebildet sein, die mittels eines Fluidstroms pneumatisch oder hydraulisch antreibbar ist.

Entsprechend kann gemäß der Erfindung auch vorgesehen sein, dass der Motor mittels einer Leitung mit einer Energiequelle verbunden ist. Eine solche Leitung kann entsprechend den obigen Ausführungen entweder eine elektrische Leitung oder eine Fluidleitung sein, die den im Stützrohr angeordneten Motor mit einer Flüssigkeit oder einem Gasstrom für den Turbinenantrieb versorgt.

Eine solche Leitung kann sich vom Motor aus dem Stützrohr heraus in Axialrichtung entweder entlang eines Blutgefäßes / der Aorta bis zu einer entsprechenden Schleuse und von dort zu einer Energiequelle erstrecken, die innerhalb oder außerhalb des Patientenkörpers angeordnet sein kann. Die Leitung kann sich jedoch auch vom Motor aus dem Stützrohr heraus in eine Herzkammer und von dort transapikal durch eine Durchführung aus dem Herzen heraus zu einer Energiequelle innerhalb oder außerhalb des Patientenkörpers erstrecken.

Beide Möglichkeiten der Leitungsführung sind physiologisch möglich und bieten je nach der Methode der Implantation des Pumpensystems unterschiedliche Vorteile.

Die Erfindung bezieht sich außer auf ein Pumpensystem der oben beschriebenen Art auch auf einen Hohlkatheter mit einem in diesem angeordneten, radial komprimierten Stützrohr sowie mit einer in dem Stützrohr fixierten, einen Rotor und ein Pumpengehäuse aufweisenden Blutpumpe. Ein solcher Hohlkatheter kann industriell vorgefertigt und vorbereitet sein und ein radial komprimiertes Stützrohr mit einer in diesem befindlichen Blutpumpe enthalten, die fertig sterilisiert und zur Implantation vorbereitet ist. Dabei kann die Blutpumpe entweder ebenfalls radial komprimiert oder auch innerhalb des Stützrohrs nicht komprimiert sein.

Ein entsprechender Hohlkatheter kann auch kurz vor einer Implantation durch Einziehen eines komprimierten Stützrohrs mitsamt einer entsprechend darin enthaltenen Blutpumpe vorbereitet werden.

Ein Hohlkatheter für die oben genannten Anwendungen weist beispielsweise einen Außendurchmesser auf, der das Durchschieben durch eine Aortenklappe mit einem Spalt erlaubt, so dass auch während des Implantationsvorgangs die physiologische Strömung im Bereich zwischen dem Hohlkatheter und der Aortenklappe nur wenig behindert wird.

Die Erfindung bezieht sich zudem auf ein Portsystem zum Implantieren eines Blutpumpensystems in den Apex des linken Ventrikels des Herzens mit einer ersten Katheterdurchführung sowie einem durch die Durchführung axial verschiebbaren Hohlkatheter. Der Hohlkatheter selbst entspricht der oben beschriebenen Gestaltung.

Vorteilhafterweise wird in einer minimalinvasiven Operation zunächst die Durchführung in einer Herzkammerwand appliziert, die der Aortenklappe in etwa gegenüberliegt (d.h. im Apex), so dass der Hohlkatheter durch die Durchführung in die Herzkammer hinein auf die Aortenklappe zu und in diese hinein geschoben werden kann. Dort wird das Stützrohr appliziert und der Hohlkatheter langsam zurückgezogen. Das Stützrohr gibt beim Herausschieben aus dem Hohlkatheter zunächst die Halteanker frei, die das Stützrohr in axialer Richtung innerhalb des Aortenklappensinus verankern. Darauf werden die Stützdrähte freigegeben, die eine radiale Fixierung durch Verklemmen an der Aortenwand bewirken, wobei sie die Aortenklappentaschen gegen die Aortenwand drücken.

Der Hohlkatheter kann dann durch die Durchführung zurückgezogen werden, wobei beispielsweise die Energieversorgungsleitung durch den zurückgezogenen Hohlkatheter freigegeben wird, die durch die Herzkammer und durch die Durchführung aus dem Herzen hinaus zu einer Energiequelle führt. Die Durchführung in der Herzwand kann dann, beispielsweise durch eine Membran mit einer die Energieleitung aufnehmenden Öffnung und insbesondere einer Manschettendichtung, derart verschlossen werden, dass sie um die durchgeführte Energieleitung herum abgedichtet ist.

Dementsprechend bezieht sich ein vorteilhaftes Verfahren zum Einbringen eines Pumpensystems an einen Einsatzort darauf, dass zunächst ein Hohlkatheter mit einem radial komprimierten Stützrohr sowie einer in diesem angeordneten Pumpe mit einem Rotor und einem Pumpengehäuse durch eine Durchführung geführt, darauf das Stützrohr aus dem Hohlkatheter herausgeschoben und derart expandiert wird, dass ein Ringspalt zwischen dem Stützrohr und dem Pumpengehäuse entsteht. Gegenstand der vorliegenden Patentanmeldung ist neben dem genannten Verfahren auch eine Vorrichtung zur Durchführung des Verfahrens bzw. auch einzelne Elemente einer solchen Vorrichtung, die zur Durchführung eines oben beschriebenen Verfahrens dienen.

Zusammenfassend kann also gesagt werden, dass die vorliegende Patentanmeldung ein implantierbares Pumpensystem, insbesondere ein transapikal implantierbares Pumpensystem, sowie einen Hohlkatheter und ein Portsystem zum Implantieren eines oben beschriebenen Blutpumpensystems betrifft. Außerdem wird auf ein entsprechendes Verfahren zum Einbringen eines Pumpensystems an einen Einsatzort Bezug genommen. Zusammenfassend sei außerdem gesagt, dass sich die genannten Gegenstände auf Anwendungen im menschlichen oder tierischen Körper beziehen können. Generell wird hiermit eine neue Art von VADs (Ventricular Assist Devices) vorgestellt. Diese sind entweder als LVAD (Left Ventricular Assist Device) oder als RVAD (Right Ventricular Assist Device) einsetzbar. Das Pumpensystem ist auch für die vollständige intravasale Implantation vorgesehen. Auf diese Weise wird durch die Kombination einer Strömung durch das erfindungsgemäße Pumpengehäuse einerseits und den Ringspalt im Pumpensystem andererseits eine nahezu physiologische Blutströmung ermöglicht, wodurch auch auf extravasale Blutwege verzichtet werden kann.

Im Folgenden wird die Erfindung anhand eines Ausführungsbeispiels in mehreren Figuren gezeigt und nachfolgend erläutert. Es zeigt
- Fig. 1: schematisch ein Stützrohr mit einer darin angeordneten Pumpe in dreidimensionaler Ansicht,
- Fig. 2: einen Querschnitt durch ein radial komprimiertes Stützrohr mit einer darin angeordneten Pumpe,
- Fig. 3: einen Querschnitt durch ein radial expandiertes Stützrohr mit einer darin angeordneten Pumpe,
- Fig. 4: eine Draufsicht auf den Ringspalt zwischen einem Stützrohr und einer Pumpe in einem segmentartigen Ausschnitt,
- Fig. 5: einen Längsschnitt durch ein Stützrohr mit einem Teil einer Pumpe und einer Ventileinrichtung,
- Fig. 6: eine Ansicht eines Teils einer Ventileinrichtung in radialer Richtung bezüglich des Stützrohrs,
- Fig. 7: die Anordnung eines Portsystems in einem menschlichen Herzen,
- Fig. 8: ein Portsystem mit einem Hohlkatheter und einem im Bereich der Aortenklappe eines Herzens eingebrachten Pumpensystem,
- Fig. 9: das Pumpensystem aus Fig. 8 mit expandiertem Stützrohr,
- Fig. 10: das Pumpensystem aus Fig. 9 nach Zurückziehen des Hohlkatheters sowie
- Fig. 11: eine Draufsicht auf das Stützrohr sowie eine Ventileinrichtung.

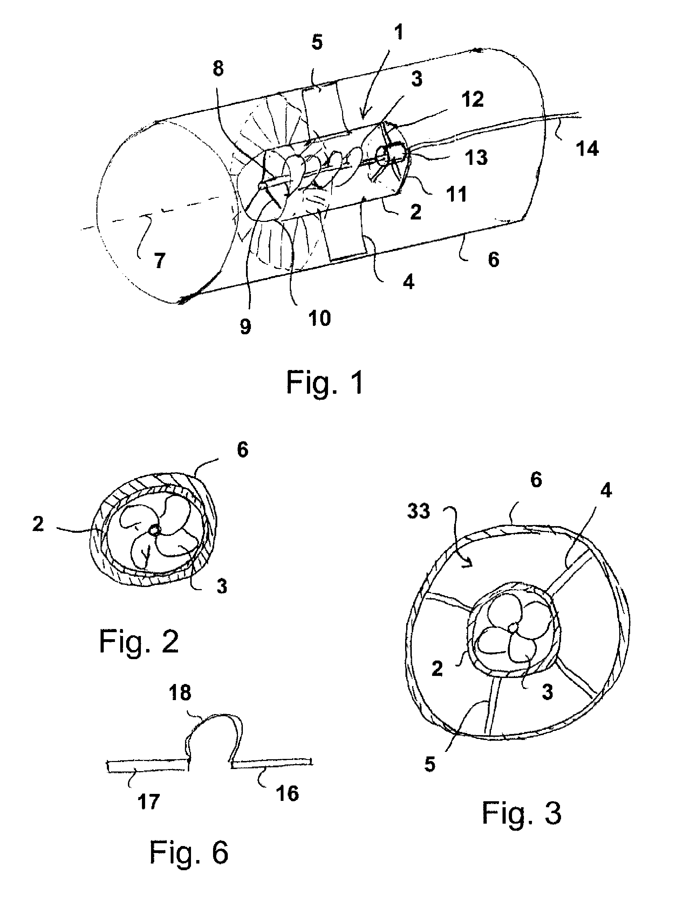

**Figur 1** zeigt in einer dreidimensionalen Ansicht eine Blutpumpe 1, die ein Pumpengehäuse 2 sowie einen Rotor 3 aufweist und mittels Stegen 4, 5 in einem Stützrohr 6 in Form eines Stents gehalten ist.

Das Pumpengehäuse 2 ist zylindrisch bzw. rotationssymmetrisch bezüglich einer Achse 7 aufgebaut, die auch mit der Mittelachse der Welle 8 des Rotors 3 zusammenfällt. Die Mittelachse 7 bezeichnet außerdem im Weiteren die Axialrichtung der Anordnung. Das Stützrohr 6 ist ebenso wie das Pumpengehäuse 2 rotationssymmetrisch aufgebaut und umgibt dieses konzentrisch.

Innerhalb des Pumpengehäuses 2 ist die Welle 8 des Rotors 3 mittels Stegen 9 in dem Pumpengehäuse 2 fixiert. Die Stege 9 sind an einem ersten Ende 10 des Pumpengehäuses 2 angeordnet. Am zweiten Ende 11 des Pumpengehäuses 2 sind weitere Stege 12 angeordnet, die einen Motor 13, der als Elektromotor ausgebildet ist, zentrisch im Pumpengehäuse fixieren. Die Welle des Motors 13 ist mit der Welle 8 des Rotors 3 verbunden oder identisch. Alternative Mittel zur Lagerung und zum Antrieb des Rotors sowie die Anordnung von Vor- oder Nachleiträdern sind ebenfalls Gegenstand des vorliegenden Schutzrechts.

Der Motor 13 wird mittels einer Leitung 14 mit elektrischer Energie versorgt und treibt den Rotor 3 an. Der Rotor 3 weist ein oder mehrere Förderelemente in Form von Schaufeln auf, die bei Rotation um die Mittelachse 7 eine Flüssigkeit in Axialrichtung fördern.

Sowohl der Rotor 3 als auch das Pumpengehäuse können radial komprimierbar sein, so dass sie beim Transport an den Einsatzort einen geringeren Raum einnehmen als im Betrieb. Das Pumpengehäuse und der Rotor können jedoch auch starr aufgebaut sein. Das Stützrohr 6 kann ebenfalls radial komprimierbar aufgebaut sein, insbesondere dann, wenn es nach Art eines Stents als faltbares Drahtgeflecht ausgebildet ist. In diesem Fall kann zum Transport das Stützrohr 6 zumindest so weit radial komprimiert werden, dass es das Pumpengehäuse 2 eng umgibt.

Dieser Zustand ist näher in **Figur 2** in einer Querschnittsdarstellung gezeigt. Dort ist das Stützrohr 6 in radial komprimierter Form direkt das Pumpengehäuse 2 umgebend dargestellt. Das Pumpengehäuse 2 ist nicht radial komprimiert und umgibt den ebenfalls nicht komprimierten Rotor 3.

In diesem Zustand kann das Pumpensystem in einfacher Weise und mit geringem operativem Aufwand bei einem Patienten an den Einsatzort gebracht werden. Hierzu kann es, wie weiter unten noch näher erläutert, zunächst in einen Hohlkatheter eingebracht und dann mit dem Hohlkatheter verschoben werden.

**Figur 3** zeigt im Querschnitt ein Pumpensystem mit einem nicht komprimierten Stützrohr 6, das konzentrisch ein Pumpengehäuse 2 mit einem Rotor 3 unter Bildung eines Ringspalts 33 umgibt. Weiterhin sind die Stege 4, 5 dargestellt, die derart faltbar sind, dass sie die radiale Kompression des Stützrohrs 6 nicht behindern. Die Stege 4, 5 können beispielsweise aus einem federelastischen oder auch aus einem biegeschlaffen Material bestehen, so dass sie bei der Fixierung des Pumpengehäuses 2 im Stützrohr 6 nur auf Zug beansprucht werden.

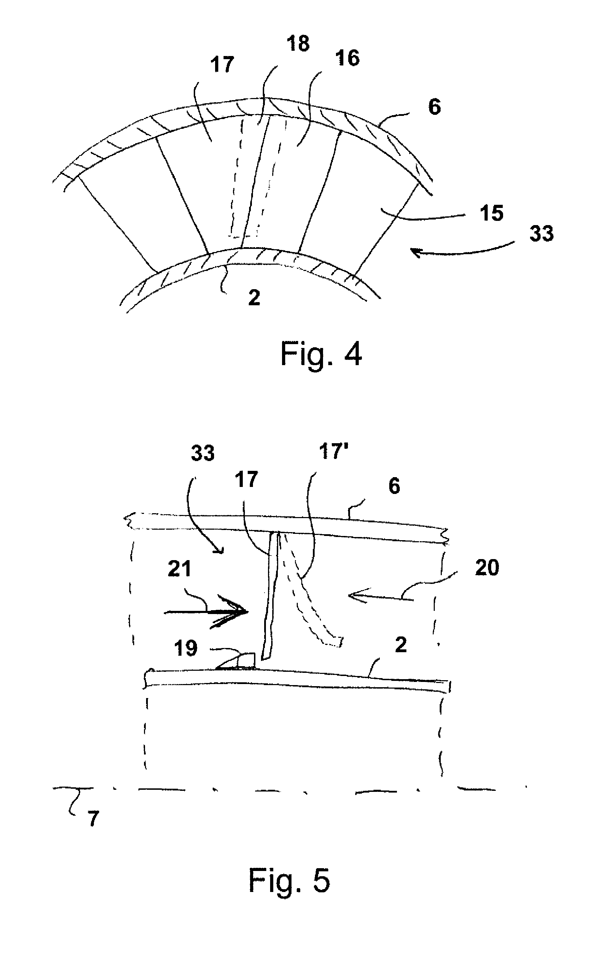

In **Figur 4** ist ein Segment des Stützrohrs 6 im Querschnitt dargestellt, gemeinsam mit einem Segment des Pumpengehäuses 2. In dem Ringspalt 33 zwischen dem Stützrohr und dem Pumpengehäuse ist eine Ventileinrichtung dargestellt, die mehrere Klappensegmente 15, 16, 17 aufweist, wobei die Klappensegmente an einem der beiden Teile, also entweder am Stützrohr 6 oder am Pumpengehäuse 2 scharnierartig befestigt sind und in Axialrichtung ausschwenken können, um den Ringspalt für eine Fluidströmung freizugeben.

Sind die Klappensegmente senkrecht zur Mittelachse 7 ausgerichtet, so liegen sie eng aneinander und versperren den Ringspalt vollständig.

Zwischen ihnen können Klappentaschen vorgesehen sein, von denen eine beispielhaft in **Figur 6** dargestellt ist. Dort sind, in radialer Richtung betrachtet, zwei Klappensegmente 17, 16 mit einer Klappentasche 18 dargestellt, die nach Art eines Filmgelenks die beiden Klappensegmente 16, 17 zumindest über einen Teil ihrer Länge, insbesondere jedoch auch über die gesamte Länge, miteinander flexibel verbindet. Die Klappentasche 18 ist in Fig. 4 gestrichelt dargestellt.

In **Figur 5** ist gezeigt, dass in der Ruheposition, vollständig ausgezogen dargestellt, die Segmente 17 senkrecht zur Mittelachse 7 zwischen dem Stützrohr 6 und dem Pumpengehäuse 2 ausgerichtet sind. In dieser Position können die Klappensegmente 17 an einem ringförmigen Anschlag 19 anschlagen, der außen konzentrisch am Pumpengehäuse 2 befestigt ist. Damit setzen die Segmente 17 einer Strömung in Richtung des Pfeils 20 Widerstand entgegen. In der entgegengesetzten Richtung, angedeutet durch den Pfeil 21, wird eine Strömung durchgelassen, indem sie die Segmente 17 in die gestrichelt dargestellte ausgelenkte Position 17' bewegt und damit den Ringspalt 33 öffnet.

Somit wird für den Bereich des Ringspalts 33 ein Rückschlagventil realisiert, das eine Flüssigkeitsströmung, insbesondere eine Blutströmung, nur in einer Strömungsrichtung durchlässt und in der entgegengesetzten Strömungsrichtung sperrt, während im zentrischen Teil des Pumpensystems, zwischen den Enden 10, 11 des Pumpengehäuses 2, die Strömung ausschließlich durch den Antrieb mittels des Pumpenrotors bestimmt wird. Es ist jedoch auch denkbar, auch im Bereich des Pumpengehäuses die Strömung mittels eines gesonderten Rückschlagventils zu steuern.

Für das Rückschlagventil ist eine andere Ausführungsform mit einer Segelklappe, wie sie für den Aortenklappenersatz bekannt ist, auch denkbar. Statt der bekannten drei Segel können auch mehr Segel verwendet werden.

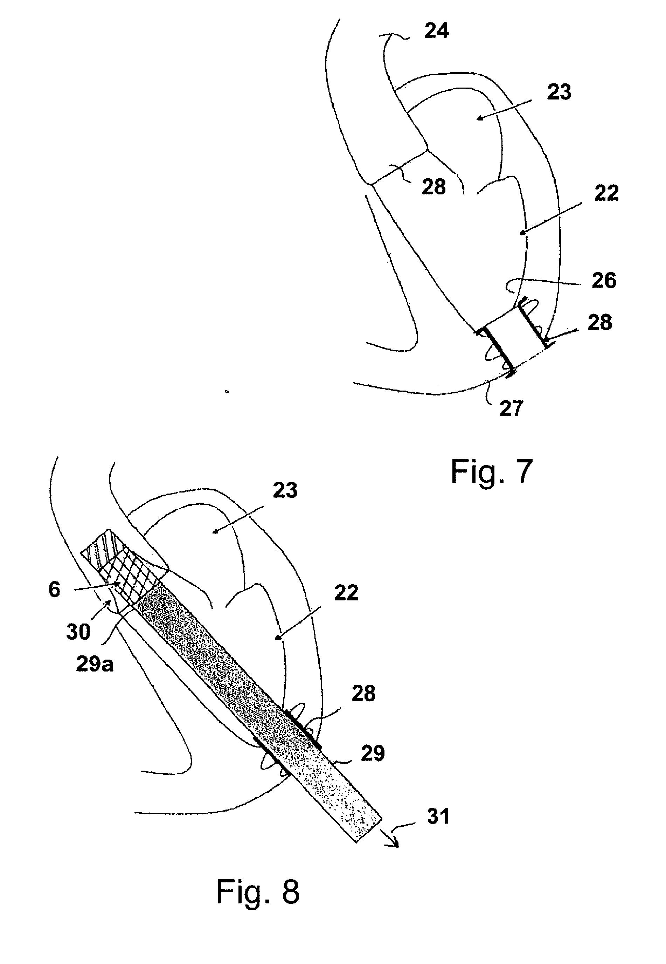

**Figur 7** zeigt als typischen Einsatzort für das erfindungsgemäße Pumpensystem ein menschliches Herz, und zwar genauer den linken Ventrikel 22, den linken Vorhof 23, die aufsteigende Aorta 24 und den Aortenklappenbereich 25.

Gegenüber dem Aortenklappenbereich 25 ist im Bereich des Apex 26 in die Herzwand 27 eine Durchführung 28 eingesetzt. Die Durchführung kann beispielsweise als Rohrstutzen mit zwei beiderseits der Herzwand 27 radial außen auskragenden Flanschen ausgebildet sein. Sie weist einen Verschlussmechanismus auf, damit sie nach der Benutzung zum Wiederherstellen der Funktionsfähigkeit des linken Ventrikels verschlossen werden kann.

Im Bereich der Aortenklappe 25 soll ein Linksherzunterstützungssystem in Form des erfindungsgemäßen Pumpensystems platziert werden.

In **Figur 8** ist gezeigt, dass von der der Aorta gegenüberliegenden Seite des Herzens her ein Hohlkatheter 29 durch die Durchführung 28 in den linken Ventrikel 22 ein- und durch diesen hindurchgeschoben ist, so dass das distale Ende 29a des Hohlkatheters mit dem komprimierten Stützrohr und der Blutpumpe im Bereich der Aortenklappe platziert ist. In dieser Position können die Halteanker 30 beim schrittweisen Zurückziehen des Hohlkatheters sich von dem Stützrohr 6 lösen und sich im Bereich des Aortensinus verankern.

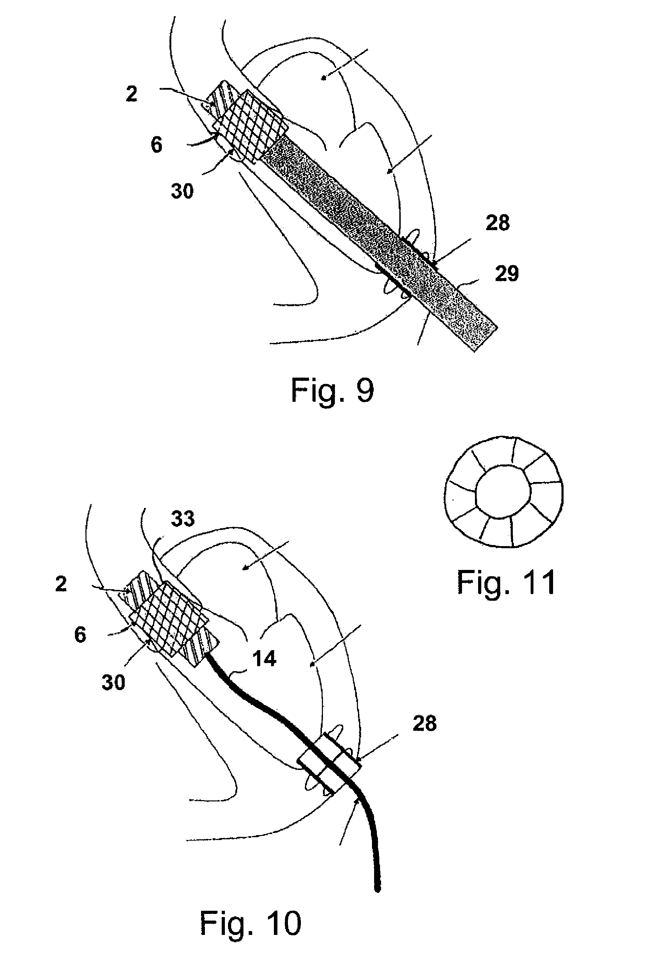

Wird der Hohlkatheter 29 weiter in Richtung des Pfeils 31 zurückgezogen, so werden Teile der Klappentaschen der Aortenklappe an die Aortenwand gedrückt. Gibt der Katheter das Stützrohr 6 in **Figur 9** vollends frei, kann es sich weiter radial ausdehnen und die Klappentaschen vollends an die Aortenwand drücken und sich dort verklemmen.

Es entsteht dabei der oben beschriebene Ringspalt 33 zwischen dem Stützrohr 6 und dem Pumpengehäuse 2. In dem dargestellten Beispiel ragt das Pumpengehäuse 2 axial über das Stützrohr hinaus. Das Pumpengehäuse kann auf einer Seite axial über das Stützrohr 6 hinausragen oder, wie in dem dargestellten Beispiel und wie in **Figur 10** vollends ersichtlich, auf beiden Seiten. Das Stützrohr kann allerdings auch länger ausgebildet sein als das Pumpengehäuse.

Die Länge des Pumpengehäuses 2 ist im hier dargestellten Fall deutlich größer als die Länge des Stützrohrs 6. Wird der Hohlkatheter 29 gegenüber der in Fig. 9 dargestellten Position noch weiter zurückgezogen und durch die Durchführung 28 hindurch entfernt, so verbleibt die Energieleitung 14, die von dem Motor des Pumpenrotors durch den linken Ventrikel und die Durchführung 28 aus dem Herzen hinausführt und mit einer Energiequelle verbunden ist, die entweder innerhalb des Patientenkörpers implantiert oder außerhalb des Patientenkörpers positioniert ist. Die Durchführung 28 kann so weit wieder verschlossen werden, dass sie um die Leitung 14 herum das Herz abdichtet.

Im Folgenden kann einerseits entsprechend der physiologischen Funktion des Herzens Blut durch den Ringspalt 33 zwischen dem Pumpengehäuse 2 und dem Stützrohr 6 ausgeworfen und in die Aorta transportiert werden, wobei in der Niederdruckphase des Herzens das Rückschlagventil wie oben beschrieben den Ringspalt abdichtet. Die zentrale in dem Stützrohr 6 angeordnete Pumpe fördert darüber hinaus mittels des rotierenden Rotors ständig Blut aus dem linken Ventrikel in die Aorta. Um ein physiologisch gewohntes Strömungsbild zu erzeugen, kann die Leistung der Pumpe rhythmisch entsprechend der pulsierenden Förderleistung des Herzens moduliert oder auch konstant gehalten werden.

Bei dem beschriebenen Pumpensystem werden weder im Ansaugbereich noch im Ausstoßbereich Kanülen benötigt, so dass die insgesamt mit dem Blut benetzte Fläche gering gehalten werden kann. Zudem ist eine nahezu physiologische, pulsierende Gestaltung der Blutströmung in der Aorta möglich.

Das Einbringen des Pumpensystems in das Herz eines Patienten kann mit geringem operativem Aufwand transapikal oder auch durch ein Blutgefäß geschehen. Perioperative Risiken sind somit ebenfalls minimiert.

## Patentansprüche

1. Transapikal implantierbares Pumpensystem zur Förderung von Blut innerhalb eines Patientenkörpers mit einer Blutpumpe (2, 3, 8), die eine Flüssigkeit in einer Axialrichtung (7) fördert und die einen rotierend antreibbaren Rotor (3) sowie ein den Rotor umgebendes Pumpengehäuse (2) aufweist, wobei das Pumpengehäuse in einem Stützrohr (6) angeordnet und gehalten ist, wobei zwischen dem Stützrohr und dem Pumpengehäuse ein Ringspalt (33) gebildet ist.

2. Pumpensystem nach Anspruch 1, **gekennzeichnet durch** eine Ventileinrichtung (16, 17, 18), die im Ringspalt angeordnet ist und die in einer ersten Flussrichtung eine Flüssigkeit in Axialrichtung (7, 21) den Ringspalt (33) passieren lässt und den Ringspalt (33) bezüglich der entgegengesetzten zweiten Flussrichtung (20) sperrt.

3. Pumpensystem nach Anspruch 1 und 2, **dadurch gekennzeichnet, dass** das Stützrohr (6) so ausgebildet ist, dass es während der Implantation in einen Patientenkörper die Klappentaschen der Aortenklappe in die Aortenwand drückt und sich selbst an dieser Position fixiert.

4. Pumpensystem nach Anspruch 1, 2 oder 3, **dadurch gekennzeichnet, dass** das Stützrohr (6) in Radialrichtung expandierbar ist.

5. Pumpensystem nach Anspruch 1 oder einem der folgenden, **dadurch gekennzeichnet, dass** das Stützrohr (6) als Stent ausgebildet ist.

6. Pumpensystem nach Anspruch 1 oder einem der folgenden, **dadurch gekennzeichnet, dass** das Stützrohr (6) mit Halteankern (30) zur axialen Positionierung versehen ist.

7. Pumpensystem nach Anspruch 1 oder einem der folgenden, **dadurch gekennzeichnet, dass** das Stützrohr (6) mit Stützdrähten zur radialen Verankerung versehen ist.

8. Pumpensystem nach Anspruch 1 oder einem der folgenden, **dadurch gekennzeichnet, dass** das Stützrohr (6) ein faltbares Drahtgeflecht aufweist.

9. Pumpensystem nach Anspruch 1 oder einem der folgenden, **dadurch gekennzeichnet, dass** die Blutpumpe (2, 3, 8) einen in Axialrichtung (7) Blut fördernden Rotor (3) aufweist, der im Pumpengehäuse (2) in wenigstens einem Lager, insbesondere in zwei Lagern, drehbar gelagert ist.

10. Pumpensystem nach Anspruch 1 oder einem der folgenden, **dadurch gekennzeichnet, dass** die Blutpumpe (2, 3, 8) im Bereich des Stützrohres (6), insbesondere innerhalb des Stützrohres (6), einen Motor (13) aufweist.

11. Pumpensystem nach Anspruch 10, **dadurch gekennzeichnet, dass** der Motor (13) mittels einer Leitung (14) mit einer Energiequelle verbunden ist.

12. Pumpensystem nach Anspruch 11, **dadurch gekennzeichnet, dass** die Leitung (14) sich in Axialrichtung (7) aus dem Stützrohr (6) heraus erstreckt.

13. Hohlkatheter (29) mit einem in diesem angeordneten, radial komprimierten Stützrohr (6) sowie mit einer in dem Stützrohr fixierten, einen Rotor (3) und ein Pumpengehäuse (2) aufweisenden Blutpumpe.

14. Portsystem zum Implantieren eines Blutpumpensystems in den Apex des linken Ventrikels des Herzens mit einer ersten Katheterdurchführung (28) sowie mit einem durch die Durchführung (28) axial verschiebbaren Hohlkatheter (29) gemäß Patentanspruch 13.

15. Portsystem nach Anspruch 14, **gekennzeichnet dadurch, dass** die Durchführung (28) ein Ventil beinhaltet, welches so gestaltet ist, dass es den Port vor dem Vorschieben und nach dem Zurückziehen des Hohlkatheters (29) gegen Herauslaufen von Blut abdichtet.

16. Verfahren zum Einbringen eines Pumpensystems an einen Einsatzort, bei dem zunächst ein Hohlkatheter (29) mit einem radial komprimierten Stützrohr (6) sowie einer in diesem angeordneten Pumpe (2, 3, 8) mit einem Rotor (3) und einem Pumpengehäuse (2) durch eine Durchführung (28) geführt, darauf das Stützrohr (6) aus dem Hohlkatheter (29) herausgeschoben und derart radial expandiert wird, dass ein Ringspalt (33) zwischen dem Stützrohr (6) und dem Pumpengehäuse (2) entsteht.

17. Pumpensystem nach einem der Ansprüche 1 bis 12, **dadurch gekennzeichnet, dass** die Pumpe mitsamt Stützrohr so gestaltet ist, dass dieses im Bereich der Aortenklappe implantierbar ist und die Aortenklappe ersetzt.
